# EUROPEAN PATENT SPECIFICATION

(11) **EP 1 200 588 B1**
(45) Date of publication and mention of the grant of the patent: **18.10.2006**
(21) Application number: 00949843.7
(22) Date of filing: 07.08.2000
(51) Int. Cl.: C12N 15/12, C12N 15/62, C07K 14/47, G01N 33/68, C12N 1/21

(54) **SINGLE-CHAIN POLYPEPTIDES COMPRISING TROPONIN I N-TERMINAL FRAGMENTS AND TROPONIN C**
EINKETTIGE POLYPEPTIDE ENTHALTEND N-TERMINALE TROPONIN I FRAGMENTE UND TROPONIN C
POLYPEPTIDES A CHAINE UNIQUE COMPRENANT DES FRAGMENTS N-TERMINAL DE TROPONINE I ET DE TROPONINE C

(30) Priority: 05.08.1999 US 368819
(43) Date of publication of application: 02.05.2002
(73) Proprietor: SPECTRAL DIAGNOSTICS INC., Toronto, Ontario M9C 1C2 (CA)
(72) Inventor: SHI, Qinwei, Etobicoke, Ontario M8Z 5V3 (CA); LIU, Shigui, Mississauga, Ontario L5A 1B6 (CA); LING, Mingfu, Etobicoke, Toronto M9B 3V9 (CA)
(74) Representative: Witz, Michael
(86) International application number: PCT/IB2000/001105
(87) International publication number: WO 2001/011043

(56) References cited:
- WO-A-98/54218
- WO-A-98/54219
- WO-A-99/31235
- SHI, Q.L.: "Recombinant single-chain cardiac troponin I-C polypetide: an ideal stable control material for cardiac troponin I immunoassays" CLINICAL INDUSTRY, vol. 45, June 1999 (1999-06), pages A53-A54, XP000941774

## Description

### FIELD OF THE INVENTION

This invention relates to recombinantly-expressed, single-chain polypeptides comprising an N-terminal fragment of troponin I linked to troponin C, and their corresponding genetic sequences.

### BACKGROUND OF THE INVENTION

Early and accurate assessment of suspected acute myocardial infarction is critically dependent on the sensitive and specific detection and quantitation in blood, serum or plasma of released cardiac muscle intracellular components in order to distinguish a potentially lethal event in need of emergency measures from non-life threatening conditions such as angina and non-cardiac chest pain such as dyspepsia. Early electrocardiographic changes are neither adequately specific nor sensitive, and the medical profession has come to rely on serum biochemical markers of cardiac tissue injury for early diagnosis. Initially, the serum markers creatine kinase (CK) and specifically the cardiac CK-MB isoform were used, and subsequently myoglobin as a more sensitive early indicator of cardiac damage. More recently, cardiac troponin complex and its subunits have come to be preferred as markers of myocardial damage because of their high specificity. These tests in combination, along with other markers of skeletal muscle damage, provide a high degree of diagnostic accuracy. If performed in the emergency room, an early and accurate diagnosis of myocardial damage offers great advantage to a suspected heart attack victim.

Diagnostic tests employing cardiac markers are described, for example, in U.S. Patent Nos. 5,604,105 and 5,290,678. These and other procedures offer the rapidity of diagnosing myocardial infarction in the emergency room setting and offer significant medical benefit for patients. Diagnostic tests in which the level of troponin subunits or complexes is measured in bodily fluids frequently utilize purified troponin subunits or complexes as antigens for the preparation of antibodies used in the assay procedure, as well as the purified subunits or complex used as controls and calibrators in performing the assays. Assay calibrators are used to prepare a series of dilutions by which a standard curve across the operating range of an assay is prepared; assay controls are used to confirm that an assay is operating properly by ensuring that the assayed value of pre-determined samples fall within an acceptable range established for each assay. In order for the assay to be calibrated properly, the troponin controls and calibrators must remain stable and in a form which is immunodetectable by the antibody.

Troponin is a muscle protein integrally involved in the calcium-dependent regulation of muscle contraction. Troponin exists in both cardiac and skeletal muscle as a non-covalently-bound complex of three subunits, the isoforms troponin C, the calcium-binding subunit, troponin I, the inhibitory subunit, and troponin T, which locates the troponin complex on tropomyosin. In vitro under the proper conditions, the troponin subunits will spontaneously associate to form non-covalently-bound complexes, e.g., troponin I and C, and troponin I, C, and T. Differences exist between the amino acid sequences of the cardiac muscle and skeletal muscle troponin isoforms.

Upon cardiac muscle injury and necrosis, troponin leaks from heart tissue into circulation, where its sensitive detection can help diagnose a heart attack. The amino acid sequence differences between the cardiac and skeletal muscle isoforms of the troponin subunits are exploited in diagnostic tests which specifically measure the cardiac isoform of the troponin subunits and complexes. Diagnostic tests for cardiac troponin I are available.

Numerous troponin preparations from both natural and recombinant sources have been described that contain troponin I together with troponin C. Malnic and Reinach (1994, Eur. J. Biochem., v. 222, pp. 49-54) produced a recombinant complex *in vivo* by cloning all three chicken skeletal muscle troponin subunits into one or more expression plasmids. Within the expression vector each troponin gene had its own promoter, and the proteins were expressed within the bacterium as individual troponin subunits, which subsequently formed complexes within the bacterium. Fujita-Becker et al. (1993, J. Biochem., v. 114, pp. 438-444) described the reconstitution of rabbit skeletal troponin complex from recombinant subunits expressed in *E. coli.* None of these recombinant products has been demonstrated to have adequate stability for use as a diagnostic test standard or calibrator. As mentioned above, even the complexes of troponin subunits are not stable and will not remain intact and bound together in solution to any great extent.

Troponin I is inherently of poor structural stability, and is subject to proteolytic cleavage by proteases present in biological samples. A troponin I material prepared in a bodily fluid matrix is thus subject to conformational alteration and degradation and is unsuitable as a calibrator or control. Furthermore, the inherently more stable troponin complexes are known to dissociate on storage and dilution, and thus become susceptible to proteolytic degradation. In the instance of troponin I, it must be complexed with troponin C in order to help maintain its conformational structure and stability; however, because of the nature of the affinity of the subunits in the complex, the fraction of the troponin I present in the form of a complex is concentration dependent. This limits its utility as an assay calibrator or control. The extent of interactions between the subunits may be calculated from the dissociation constant, K_{d} [for example, as reported in Biochemistry 33:12729 [1994]]. By calculation and experimental measurement, only a limited amount of troponin I is bound to troponin C over the range that would be found in patient serum samples, and thus the levels at which calibrators and controls must be used. For example, at 50 ng/ml, the upper range of most troponin I assays, only 10% of the troponin I is bound to troponin C when the two subunits are present at a ratio of 1:1. With up to 10-fold more troponin C to troponin I, and in the presence of divalent cations, a claim (Larue et al., U.S. Patent 5,583,200) to the stability of the complex in the cold was minimal, i.e., "at least one day." Maintaining higher concentrations of the complex decreases the degree of dissociation; however, after dilution to the level necessary to calibrate an assay, the subunits dissociate and become immunologically unstable. Dissociation then subjects the subunits to proteolytic attack, further reducing the utility of such calibrators and controls. As described in the above-identified application, stable N-terminal fragments of troponin I, optionally with an intact N-terminus, are described which are immunostable and are useful as calibrators and controls for optimized troponin I assays.

Furthermore, as described in copending U.S. Patent 6,077,676, a stable troponin preparation for assay and other uses has been described which comprises troponin I and troponin C on a single polypeptide chain, prepared as a recombinant construct and expressed in a bacterial expression system as a single polypeptide. As noted above, association of troponin I and troponin C protects the troponin I molecule from conformational changes and proteolytic attack; in the single-chain construct, the troponin subunits cannot dissociate, and thus the troponin I is immunostable and resistant to proteolytic degradation.

WO 99/31235 relates to single-chain polypeptides and their genetic sequences comprising human cardiac troponin I and troponin C. The single-chain polypeptide may be expressed recombinantly, and a linker peptide may be interposed between the troponin sequences. A linker peptide of about 6 to about 30 amino acids is preferred. The single chain polypeptide has utility as a control or calibrator for troponin assays, for the purification of troponin subunits and as an antigen for the preparation of antibodies.

WO 98/54219 discloses stabilized compositions for use in clinical assays for troponin, such as a composition for use in an assay for the determination of the presence or concentration of cardiac troponin I, the composition comprising: a complex of cardiac troponin I or a fragment thereof and troponin C or a fragment thereof wherein the cardiac troponin I is covalently coupled to the troponin C. The covalent coupling is said to be cross-linking between these proteins introduced by means of suitable coupling agents.

It is towards the development of an immunostable and immunodetectable troponin I composition that the present invention is directed.

### GLOSSARY

"N-terminal fragment of troponin I" refers to a portion of the troponin I molecule derived from the N-terminal part of the native molecule, which may be any fragment from the N-tenninus (amino acid 1) to about the middle of the native molecule (about amino acid 110). The N-terminal fragment may or may not have the native N-terminus. A non-limiting example is an 82 amino acid fragment corresponding to amino acids 28 through 110 of native human cardiac troponin I.

"N-terminus" is the N-terminal amino acid 1 of native troponin I.

"Linker" refers to a sequence of about 5 to about 50 amino acids (and its corresponding polynucleotide sequence) interposed between the troponin I fragment and troponin C of the present invention to permit the troponin molecules to associate in a fashion similar to their association in the native non-covalent troponin I-troponin C complex.

"Spacer" refers to a short amino acid sequence of about 4 or fewer amino acids (and its corresponding polynucleotide sequence) interposed between the troponin I fragment and troponin C for the purpose of facilitating the preparation of the recombinant construct using a short restriction site.

### SUMMARY OF THE INVENTION

It is a principal objective of the present invention to provide single-chain polypeptides comprising an N-terminal fragment of troponin I and troponin C. The presence of the troponin I N-terminal fragment and troponin C on the same polypeptide chain confers conformational stability and immunostability to the product. Portions of the single-chain polypeptide comprising an N-terminal fragment of troponin I, optionally lacking the N-terminus, resemble the stable circulating form of troponin I which is partially degraded by proteinases, either at the amino end, the carboxy end or both are similar to the main serum form that is measured in a patient's blood sample for identifying the cause of chest pain and, more specifically, to diagnose a heart attack. The circulating, partially degraded form of troponin I joined to troponin C on the same polypeptide provide stable compositions substantially similar in structure to the analyte being measured and with epitopes which will combine with the analyte. They provide immunostable and immunodetectable compositions suitable for use as calibrators and controls for troponin I immunoassays, as well as for affinity purification of troponin antibodies.

The single-chain polypeptide may preferably include a linker sequence interposed between the sequences of the N-terminal troponin I fragment and troponin C. The sequence of the linker peptide is chosen so that it does not interfere with the tertiary structure of the product and therefore its aforementioned useful properties. A single-chain polypeptide in which an N-terminal fragment of troponin I and troponin C are joined, optionally through a linker peptide, provides a stable, reproducible, and easily purified material for the development of troponin assays, an antigen for preparing troponin antibodies, as well as material for use as controls and calibrators for troponin assays. In a non-limiting example of an N-terminal fragment of troponin I joined through a linker sequence to troponin C, the troponin I fragment comprises residues 28 through 110 of native troponin I, joined through a 19-amino acid linker sequence to native troponin C. In a further example, a single-chain polypeptide comprises an N-terminal fragment of troponin I, residues 28 to 110 of native troponin I, linked to the native troponin C sequence without a linker. In order to engineer the construct, a two amino acid sequence is provided between the troponin portions.

The N-terminal fragment of troponin I is lacking the N-terminus. In one embodiment, about 20 to about 30 amino acids of the N-terminal portion are absent. Thus, the N-terminal troponin I fragment of the single-chain polypeptide of the present invention provides an N-terminal troponin I fragment of about 65 to about 85 amino acids. By way of non-limiting examples, the single-chain polypeptides have amino acids 28 through 110 of native troponin I.

The single-chain polypeptide of the present invention is prepared most readily by recombinant techniques, by constructing a replicable cloning or expression vehicle such as a plasmid carrying the genetic sequence for the single-chain polypeptide, and transforming a host cell, such as *E. coli,* with the vehicle such as a plasmid, and expressing the polypeptide by the host cell. The single-chain construct may contain a linker peptide sequence between the troponin I fragment and the troponin C amino acid sequences, such sequence introduced by recombinant means. If no linker peptide is provided, an optional short sequence of about two amino acids may be provided between the troponin sequences in order to engineer the construct. Certain modifications may be made in the genetic sequence of the polynucleotide, with or without changes in the consequent amino acid sequence of the polypeptide, in order to improve the expression of the polypeptide in the host cell. These changes do not alter the properties of utilities of the single-chain polypeptide for the aforementioned purposes.

It is a further aspect of the invention to provide methods for using the single-chain polypeptides described hereinabove as calibrators or controls for troponin I assays.

It is another object of the present invention to provide a genetic sequence for a single-chain polypeptide comprising a genetic sequence of an N-terminal fragment of troponin I, lacking the N-terminus, and troponin C. The genetic sequence may also include a linker genetic sequence interposed between the genetic sequences of troponin I and troponin C. In the absence of a linker sequence, a short sequence equivalent to two amino acids may be introduced therebetween to enable the engineering of the construct. A host cell may be transformed with the replicatable cloning or expression vehicle containing the aforementioned genetic sequences. As mentioned above, certain changes to the genetic sequence of the troponins may be made in order to facilitate expression in the host cell.

It is a further object of the present invention to provide a host cell containing a cloning or expression vehicle such as a plasmid carrying the genetic sequence for a single-chain polypeptide chain comprising the genetic sequences of an N-terminal troponin I fragment and troponin C, and capable of expressing a single-chain polypeptide comprising troponin I and troponin C.

These and other aspects of the present invention will be better appreciated by reference to the following Detailed Description.

### DETAILED DESCRIPTION OF THE INVENTION

For utility as stable calibrators and controls for troponin I assays, the compositions of the present invention improve upon the inherent conformational instability and proteolytic susceptibility of free troponin I, the instability of association of the troponin I-troponin C complex, and the dissimilarity between the predominant circulating form of troponin I in a sample and the native troponin I analytes. The improvements consists of a single-chain polypeptide comprising an N-terminal fragment of human cardiac troponin I and cardiac troponin C. The troponin subunits are covalently linked through a peptide bond and reside on the same linear polypeptide, providing a stable troponin I fragment-troponin C single-chain polypeptide to meet the needs of the industry. The N-terminal fragment of troponin I in the polypeptide resembles the main form of troponin I in circulation, following partial degradation by intracellular proteinases and serum proteinases, which cleave the C-terminal portion of troponin I, leaving an intact N-terminal fragment, which may be further cleaved at the N-terminal portion. The remaining fragment of troponin I, approximately between amino acids 28 and 110, is highly resistant to further proteolytic degradation due to its primary structure. Stability of the tertiary structure of the troponin I fragment is conferred by association with troponin C. Thus, a single chain troponin I N-terminal fragment and troponin C construct provides a degradation resistant and structurally stable polypeptide, which is in a form similar to that of the main serum analyte.

The single-chain polypeptide may be prepared by recombinant techniques, and may optionally include a linker polypeptide or a spacer polypeptide sequence interposed between the troponin I and troponin C sequences.

For example, one embodiment of the troponin I fragment-troponin C single-chain polypeptide may comprise a troponin I fragment at the N-terminal portion of the polypeptide, with the C-terminus of the troponin I fragment sequence engaged in a peptide bond with the N-terminus of the troponin C sequence. In one non-limiting example, in order to engineer such a construct, a short sequence of amino acids, about 2 is provided between the troponin molecules. This permits the use of restriction endonucleases in preparing the construct. For example, the SphI endonuclease site codes for Ala Cys. In another embodiment, a construct in which the troponin I fragment is joined directly to the troponin C may be prepared by techniques such as solid-phase synthesis, of either the polynucleotide or the protein, or use of the SOEing procedure described below. In a second embodiment wherein a linker peptide sequence is interposed between the troponin I fragment and the troponin C amino acid sequences, one preferable arrangement comprises the troponin I fragment sequence at the N-terminal portion of the polypeptide, its C-terminus engaged in a peptide bond with the N-terminus of the linker peptide, and the C-terminus of the linker peptide then engaged in a peptide bond with the N-terminus of the troponin C sequence. In an example of this construct, the amino acid sequence of the linker contains 19 amino acids. It should be noted that in engineering the construct with the linker, nucleotides equivalent to an additional two amino acids is introduced at each end of the linker polynucleotide in preparing the construct. As referred to herein, the linker sequence comprises the linker sequence and the two amino acids at each end to facilitate the engineering of the product. As mentioned above, the intermediate amino acids may be omitted if alternate methods of preparing the product are used, for example, by solid phase synthesis of the polynucleotide or the amino acid sequence, by use of the SOEing method (see below), or by identifying restriction endonucleases which exactly recognize the termini of the troponin species and linker sequences being linked.

The amino acid sequences in the above example correspond to the nucleotide sequences of the cDNA coding for these polypeptides. The genetic sequence in the first example comprises the troponin I fragment genetic sequence at the 5' end of the cDNA, its 3' end followed immediately by two intermediate codons and then the 5' end of the troponin C genetic sequence. In the preferred embodiment wherein a linker is interposed between the troponin I and troponin C sequences, the 5' of the cDNA sequence begins with the troponin I genetic sequence, its 3' end followed by the 5' end of the optional interposed linker genetic sequence, and its 3' end followed by the 5' end of the troponin C genetic sequence, ending at the 3' end of the cDNA.

As described above, selection of the length and specific sequence of the optional linker polypeptide is limited only in that it must not interfere with the immunodetectability of the troponin I fragment on the single-chain polypeptide. It is believed that with a suitable linker sequence, the troponin I fragment and the troponin C of the single polypeptide chain associate with each other in a similar fashion as they do in a non-covalent troponin I-troponin C complex, and the attachment of the subunits in the single polypeptide chain maintains the conformation of the association and thus the consistent immunodetectability of the troponin. Furthermore, a troponin I fragment-troponin C complex stabilized in this manner is less susceptible to proteolytic attack in the presence of bodily fluids and other components. In this embodiment, a linker of about 6 to about 50 amino acids (and a corresponding number of codons in the cDNA) is preferred, for ease and economics of preparation.

One embodiment of the present invention provides a single-chain troponin I fragment-troponin C polypeptide with a relatively short spacer segment of about 5 to about 50 amino acids. For example, a useful linker polypeptide sequence is (Gly₄Ser)₃ which provides a flexible peptide sequence that allows the two subunits to associate. In order to construct the genetic sequence with a linker polynucleotide, an additional 2 codons at each end of the linker polynucleotide are present, which were needed in order to provide unique restriction sites to create the genetic construct of the desired single-chain polypeptide. In one example, codons corresponding to Thr-Ser at the N-terminus of the linker and Ala-Cys at the C-terminus, may be included. Thus, a suitable 19-residue linker may be prepared. The above example is only representative of the linked products of the present invention, as other linkers are suitable.

Recombinant methods may be used to prepare the DNA sequence comprising the troponin subunits and the optional linker sequence and to introduce the sequence into a host cell, and standard expression methods are used to express and purify the recombinant polypeptide. These methods are similar to those used for the preparation of fusion proteins such as that described for the two metabolically-coupled yeast enzymes, citrate synthase and malate dehydrogenase (Lindbladh et al., Biochemistry 33:11692-11698 [1994]); in the preparation of single-chain polypeptides comprising the antigen-binding site of antibodies (U.S. Patent 4,946,778); and the preparation of fusion proteins for phage display (U.S. Patent 5,516,637). These methods are known to the skilled artisan.

In the instance in which little or no linker sequence is desired, the troponin I fragment and troponin C cDNA sequences may be joined through suitable techniques known in the art such as the SOEing method using pairs of partially overlapping primers, for example, as described by Hu et al. (1996, Protein Expression and Purification 7:289-293) in which rare codons in human cardiac troponin T were replaced with synonymous major codons. These methods are well known to the skilled artisan.

The recombinant construct is prepared as an expression or cloning vehicle, or plasmid, and introduced into a host cell for expression. Methods for expression of recombinant proteins are known in the art. Once expressed, the single-chain polypeptide may be purified by standard protein purification methods.

Furthermore, the genetic sequences of the troponin I fragment and troponin C may be modified in order to improve the expression of the single-chain polypeptide in a bacterial expression system. These genetic alterations may or may not alter the amino acid sequence of the polypeptide. As is known in the art, certain rare codons present in an expression vehicle reduce expression efficiency, and by changing these codons to synonymous major codons, bacterial expression is improved (for example, as that described by Hu *et al., supra*)*.* In addition, the inclusion of a short nucleotide sequence to the 5' end of the troponin I cDNA increases bacterial expression, and provides a troponin I polypeptide with an additional six N-terminal amino acids. These optional modifications to increase bacterial expression do not detract from the utility of the single-chain polypeptide for the aforementioned purposes. The short addition to the 5'end of the cDNA to enhance expression may also be added to the N-terminus of a troponin I fragment lacking the native N-terminus, for example, the troponin I fragment described in the Examples herein.

Several troponin I assays are commercially available, all of which operate using different formats, instruments, and assay controls and calibrators. For example the Stratus(R) troponin I assay from Dade utilizes a monoclonal capture and monoclonal detector antibody. The calibrator/control material is an N-terminal peptide from human cardiac troponin I. The operating range of the assay is 0-50 ng/ml, with a sensitivity of 0.6 ng/ml and a cut-off value of 1.5 ng/ml. The Access (R) troponin I assay from Sanofi also utilizes a monoclonal capture and monoclonal detector antibody, but its calibrator/control is a complex of native cardiac troponin I and troponin C. This assay has an operating range of 0-50 ng/ml, a sensitivity of 0.03 ng/ml, and a cut-off value of 0.1 ng/ml. The Opus(R) troponin I assay from Behring utilizes polyclonal antibodies as both capture and detector, has a range of 0-300 ng/ml, a sensitivity of 1 ng/ml and a cut-off value of 2 ng/ml.

Because of the differences in the methodology and components among the above-mentioned assays, commercially-available troponin I calibrators/controls often cannot be used interchangeably among assays. For example, the Stratus(R) calibrators/controls, which use a N-terminal peptide from cardiac troponin I, are not detectable in the Access(R) and Opus(R) assays, as the latter assays' antibodies are not directed to the same N-terminal peptide portion of troponin I used for the controls/calibrators. On the other hand, the Access(R) assay controls, which are detectable in the Access(R) assay with the highest level of sensitivity and cut-off value of all three assays, measure about four times higher in the Opus(R) assay. In contrast, the Opus(R) assay controls are detected poorly by the Stratus(R) assay. These results indicate that it is difficult to interchange assay calibrators/controls between assays, and that the values provided by the controls of one manufacturer's assay can only be used in interpreting assays run on that assay. However, for the constructs of the present invention in which the C-terminal portion of troponin I is absent, assays which detect this portion of the troponin I molecule, for example, the Access assay, would not be suitable for use with the instant compositions.

A single-chain polypeptide of this invention comprising a troponin I fragment and troponin C may also be used for the purification of proteins and other substances including antibodies with an affinity for binding troponin I or troponin C. As noted above, the troponin I portion of the instant compositions is believed to resemble the major circulating form of troponin I after partial degradation by serum proteinases, and perhaps by cardiac intracellular enzymes. For example, the single-chain polypeptide of the present invention may be covalently bound to an insoluble matrix or polymer and situated in a chromatography column. A cell or tissue extract suspected of containing a material that binds troponin, or an antibody preparation raised against troponin, may be passed through the column, whereby it would adhere to the covalently-bound polypeptide. After washing the matrix, the adherent material may be eluted using a concentrated salt solution, a chaotropic agent, or other standard methods used in protein purification. Material binding to the troponin I fragment of the composition would be expected to be more suitable for the detection of the circulating form of troponin I.

A single-chain polypeptide of the present invention may also be useful for the preparation of monoclonal or polyclonal anti-troponin antibodies, using standard methods of animal immunization or hybridoma preparation. For the reasons mentioned above, resulting antibodies are expected to recognize the circulating form of troponin I, and thus improve the sensitivity of an assay made therefrom.

The single-chain polypeptide of the present invention comprising an N-terminal portion of troponin I and troponin C has utility for the preparation of sensitive troponin assays and for the calibration of such assays. By way of example, troponin I may be quantitated by following a particular analytical procedure, for example, by immunoassay, such as those described above. The troponin I level in a sample and the troponin I level in a standard comprising the single-chain polypeptide of the present invention are measured using the particular procedure, wherein the quantity of troponin I in the standard is known. The quantity of troponin I in the sample is then calculated by correlating the measured value of the sample to the measured value of the standard, based on the known quantity of troponin I in this calibrator. As will be seen from the following non-limiting examples, the single-chain polypeptide exhibits superior performance when compared to other troponin calibrators.

### Example 1

### Expression in E. coli of a single-chain human cardiac troponin I tragment-troponin C Polypeptide with a linker

cDNAs of a human cardiac troponin I fragment corresponding to amino acids 28-110 of the native protein, and troponin C cDNAs were cloned by polymerase chain reaction (PCR) using primers designed from the published cardiac troponin I cDNA sequence (Vallins et aL, FEBS Letters 270,57-61 [1990]) and the troponin C sequence (GenBank AC: X07897). The C-terminus of the troponin I fragment cDNA was linked with the N-terminus of troponin C cDNA through a synthetic linker coding for (Gly₄Ser)₃ with an unique restriction site engineered on each end. The single-chain troponin I fragment-C cDNA construct was confirmed by DNA sequencing and cloned into expression vector pET21(Novagen). *E. coli* BL21(DE3) cells, also available from Novagen, were transformed with the resulting plasmid and protein expression was verified by both SDS-PAGE and immunoassays. The single-chain polypeptide described above has a molecular weight of 29,200 Daltons.

The E. coli strain expressing the single-chain troponin I fragment-troponin C polypeptide with a linker has been deposited with the American Type Culture Collection (ATCC), 10801 University Boulevard, Manassas VA 20110-2209, and has been accorded number PTA-396.

### Example 2

### Expression in E. coli of a single-chain human cardiac troponin I fragment-troponin C polypeptide without a linker

cDNA of a human cardiac troponin I fragment corresponding to amino acids 28-110 of the native protein, and troponin C, were cloned by polymerase chain reaction (PCR) using primers designed from the published cardiac troponin I cDNA sequence (Vallins et al., FEBS Letters 270,57-61 [1990]) and the troponin C sequence (GenBank AC: X07897). The C-terminus of the troponin I fragment cDNA was linked with the N-terminus of troponin C cDNA through a SphI site which codes for Ala and Cys. The single-chain troponin I fragment-C cDNA construct was confirmed by DNA sequencing and cloned into expression vector pET21(Novagen). *E. coli* BL21(DE3) cells, also available from Novagen, were transformed with the resulting plasmid and protein expression was verified by both SDS-PAGE and immunoassays. The single-chain polypeptide described above has a molecular weight of 28,100 Daltons.

The E. coli strain expressing the single-chain troponin I fragment-troponin C polypeptide has been deposited with the ATCC and has been accorded number PTA-395.

### Example 3

### Stability and Utility of the Polypeptide in the Troponin Assay

The immunodetectability of the single-chain troponin I fragment- troponin C polypeptides described in Examples 1 and 2, a complex formed from native cardiac troponin I and troponin C, and native troponin I, were evaluated in the Stratus(R), and Opus(R) assays, following manufacturer's procedures for each assay. The results showed that the immunodetectability on a molar equivalent basis of troponin I was equivalent to or better than that of troponin I in the non-covalent complex and as native, non-complexed troponin L

### Example 4

### Stability of the Single-chain Troponin I fragment-troponin C Polypeptide

The stability of the three preparations containing troponin I or fragment used in Example 3 above was followed during incubation in serum at 37°C for various time periods by determining the molecular weight using a Western blot. Antibodies to the N-terminal portion of troponin I were used.. The preparations were (1) recombinant troponin I prepared by standard methods; (2) a non-covalently-bound complex of recombinant troponin I and recombinant C, and (3) the single-chain polypeptide comprising a troponin I fragment and troponin C with an interposed linker peptide. The non-covalently-bound complex of recombinant troponin I and recombinant troponin C was prepared by the procedure of commonly-owned US 5,834,211. Briefly, human cardiac troponin C and a modified troponin I were expressed in E. coli. The troponin I was engineered as a recombinant product with six additional N-terminal amino acid residues, to increase its expression; troponin C was expressed with its native amino acid sequence. The modified troponin I in the presence of urea was combined with troponin C, CaCl₂ and MgCl₂, and shaken gently to promote the formation of troponin I-troponin C complexes.

Results showed that the native troponin I was most susceptible to proteolytic degradation on exposure to serum. The non-covalent troponin I-troponin C complex was more stable, and the single-chain polypeptide of the present invention was most stable.

## Claims

1. A polynucleotide encoding a single-chain polypeptide comprising an N-terminal fragment of cardiac troponin I and a cardiac troponin C, wherein said N-terminal troponin I fragment consists of a sequence starting with amino acid number 20 to amino acid number 30 of native human cardiac troponin I, and extending to amino acid number 95 to amino acid number 115 of native human cardiac troponin I.

2. The polynucleotide of claim 1, wherein a linker polynucleotide coding for a linker peptide sequence of 5 to 50 amino acids is interposed between the cardiac troponin I N-terminal fragment polynucleotide and the cardiac troponin C polynucleotide.

3. The polynucleotide of claim 1 or 2, wherein said an N-terminal fragment of cardiac troponin I consists of amino acid numbers 28 -110 of native human cardiac troponin I.

4. The polynucleotide of claim 1 encoding a single-chain polypeptide consisting of:
a) a polypeptide comprising the N-terminal portion of human cardiac troponin I of 65 to 110 amino acids; and
b) a human cardiac troponin C.

5. A replicatable cloning or expression vehicle comprising a polynucleotide of any one of claims 1-3.

6. The vehicle of claim 5, which is a plasmid.

7. A host cell transformed with a vehicle of claim 5.

8. The host cell of claim 7, which is E. coli.

9. The host cell of claim 7, which is E. coli deposited with the American Type Culture Collection and accorded number PTA-395 or number PTA-396.

10. A single-chain polypeptide comprising an N-terminal fragment of cardiac troponin I and a cardiac troponin C, wherein said N-terminal fragment of troponin I consists of a sequence starting with amino acid number 20 to amino acid number 30 of native human cardiac troponin I, and extending to amino acid number 95 to amino acid number 115 of native human cardiac troponin I.

11. The polypeptide of claim 10, wherein a linker peptide sequence of 5 to 50 amino acids is interposed between the N-terminal fragment of cardiac troponin I and the cardiac troponin C polypeptide.

12. The polypeptide of claim 10 or 11, wherein said an N-terminal fragment of cardiac troponin I consists of amino acid numbers 28-110 of native human cardiac troponin I.

13. The polypeptide of any one of claims 10 - 12, wherein the linker peptide sequence comprises (Gly₄Ser)₃.

14. The polypeptide of any one of claims 10 -13, in the form of the expression product obtainable from culturing of the E. coli of claim 9.

15. A composition comprising a single-chain polypeptide of any one of claims 10 - 13 for use as a control or calibrator for a troponin I assay.

16. A method for quantitating troponin I in a sample, comprising the steps of:
i) measuring troponin I in said sample;
ii) measuring a single-chain polypeptide of any one of claims 10-13 in a standard, said standard having a known quantity of troponin I therein;
iii) correlating the troponin I measured in the sample with the known quantity of troponin I measured in the standard to provide the quantity of troponin I in said sample.

## Patentansprüche

1. Polynukleotid, das ein einkettiges Polypeptid kodiert, welches ein N-terminales Fragment des kardialen Troponin I und ein kardiales Troponin C umfasst, wobei das N-terminale Fragment von Troponin I aus einer Sequenz besteht, die bei Aminosäure Nummer 20 bis Aminosäure Nummer 30 des nativen kardialen Human-Troponin I beginnt und bis zu Aminosäure Nummer 95 bis Aminosäure Nummer 115 des nativen kardialen Human-Troponin I reicht.

2. Polynukleotid nach Anspruch 1, wobei zwischen dem Polynukleotid für das N-terminale Fragment des kardialen Troponin I und dem Polynukleotid für das kardiale Troponin C ein Verbindungspolynukleotid angeordnet ist, das eine Verbindungspeptidsequenz von 5 bis 50 Aminosäuren kodiert.

3. Polynukleotid nach Anspruch 1 oder 2, wobei das N-terminale Fragment des kardialen Troponin I aus den Aminosäuren Nummer 28 bis 110 des nativen kardialen Human-Troponin I besteht.

4. Polynukleotid nach Anspruch 1, das ein einkettiges Polypeptid kodiert, bestehend aus:
a) einem Polypeptid, das den N-terminalen Abschnitt des kardialen Human-Troponin I mit 65 bis 110 Aminosäuren umfasst; und
b) einem kardialen Human-Troponin C.

5. Replizierbares Klonierungs- oder Expressionsvehikel, das ein Polynukleotid nach einem der Ansprüche 1 bis 3 umfasst.

6. Vehikel nach Anspruch 5, das ein Plasmid ist.

7. Wirtszelle, die mit einem Vehikel nach Anspruch 5 transformiert ist.

8. Wirtszelle nach Anspruch 7, die *E. coli* ist.

9. Wirtszelle nach Anspruch 7, die *E. coli* ist, welches bei der American Type Culture Collection hinterlegt ist und dem die Nummer PTA-395 oder die Nummer PTA-396 zugeordnet ist.

10. Einkettiges Polypeptid, das ein N-terminales Fragment des kardialen Troponin I und ein kardiales Troponin C umfasst, wobei das N-terminale Fragment von Troponin I aus einer Sequenz besteht, die bei Aminosäure Nummer 20 bis Aminosäure Nummer 30 des nativen kardialen Human-Troponin I beginnt und bis zu Aminosäure Nummer 95 bis Aminosäure Nummer 115 des nativen kardialen Human-Troponin I reicht.

11. Polypeptid nach Anspruch 10, wobei zwischen dem N-terminalen Fragment des kardialen Troponin I und dem kardialen Troponin C-Polypeptid eine Verbindungspeptidsequenz von 5 bis 50 Aminosäuren angeordnet ist.

12. Polypeptid nach Anspruch 10 oder 11, wobei das N-terminale Fragment des kardialen Troponin I aus den Aminosäuren Nummer 28 bis 110 des nativen kardialen Human-Troponin I besteht.

13. Polypeptid nach einem der Ansprüche 10 bis 12, wobei die Verbindungspeptidsequenz (Gly₄Ser)₃ umfasst.

14. Polypeptid nach einem der Ansprüche 10 bis 13 in Form des Expressionsprodukts, das durch Kultivierung des *E. coli* nach Anspruch 9 erhalten werden kann.

15. Zusammensetzung, umfassend ein einkettiges Polypeptid nach einem der Ansprüche 10 bis 13 zur Verwendung als Kontrollsubstanz oder Kalibrator für ein Troponin I-Assay.

16. Verfahren zum quantitativen Bestimmen von Troponin I in einer Probe, das folgende Schritte umfasst:
i) Messen von Troponin I in der Probe;
ii) Messen eines einkettigen Polypeptids nach einem der Ansprüche 10 bis 13 in einem Standard, wobei der Standard eine bekannte Menge Troponin I enthält;
iii) Korrelieren des Troponin I, das in der Probe gemessen wurde, mit der bekannten Menge von Troponin I, die im Standard gemessen wurde, um die Menge von Troponin I in der Probe zu erhalten.

## Revendications

1. Polynucléotide qui code un polypeptide à chaîne unique comprenant un fragment N-terminal de troponine I cardiaque et de troponine C cardiaque, dans lequel ledit fragment N-terminal de troponine I consiste en une séquence qui commence de l'acide aminé numéro 20 à l'acide aminé numéro 30 de troponine I cardiaque humaine native et s'étend jusqu'à l'acide aminé numéro 95 à l'acide aminé numéro 115 de troponine I cardiaque humaine native.

2. Polynucléotide selon la revendication 1, dans lequel un codage de polynucléotide de liaison pour une séquence de petides de liaison de 5 à 50 acides aminés est interposé entre le polynucléotide de fragment N-terminal de troponine I cardiaque et le polynucléotide de troponine C cardiaque.

3. Polynucléotide selon la revendication 1 ou 2, dans lequel un fragment N-terminal de troponine I cardiaque se compose des acides aminés numéros 28 - 110 de troponine I cardiaque humaine native.

4. Polynucléotide selon la revendication 1 qui code un polypeptide à chaîne unique consistant en :
a) un polypeptide comprendant la partie N-terminal de troponine I cardiaque humaine de 65 à 110 acides aminés ; et
b) une troponine C cardiaque humaine.

5. Véhicule de clonage ou d'expression réplicable comprenant un polynucléotide selon l'une quelconque des revendications 1-3.

6. Véhicule selon la revendication 5 qui est un plasmide.

7. Cellule hôte transformée avec un véhicule selon la revendication 5.

8. Cellule hôte selon la revendication 7, qui est le E. coli.

9. Cellule hôte selon la revendication 7, qui est le E. coli déposé à la American Type Culture Collection et à laquelle est affecté le numéro PTA-395 ou le numéro PTA-396.

10. Polypeptide à chaîne unique comprenant un fragment N-terminal de troponine I cardiaque et de troponine C cardiaque, dans lequel ledit fragment N-terminal de troponine I consiste en une séquence qui commence de l'acide aminé numéro 20 à l'acide aminé numéro 30 de troponine I cardiaque humaine native et s'étend jusqu'à l'acide aminé numéro 95 à l'acide aminé numéro 115 de troponine I cardiaque humaine native.

11. Polypeptide selon la revendication 10, dans lequel une séquence de petides de liaison de 5 à 50 acides aminés est interposée entre le fragment N-terminal de troponine I cardiaque et le polypeptide de troponine C cardiaque.

12. Polypeptide selon la revendication 10 ou 11, dans lequel un fragment N-terminal de troponine I cardiaque se compose des acides aminés numéros 28-110 de troponine I cardiaque humaine native.

13. Polypeptide selon l'une quelconque des revendications 10 -12, dans lequel la séquence de peptides de liaison comprend (Gly₄Ser)₃.

14. Polypeptide selon l'une quelconque des revendications 10 -13 sous la forme du produit d'expression que l'on peut obtenir de la culture du E. coli de la revendication 9.

15. Composition comprenant un polypeptide à chaîne unique selon l'une quelconque des revendications 10-13 pour utilisation comme organe de contrôle ou calibreur pour un échantillon prélevé de troponine I.

16. Méthode de détermination de la quantité de troponine I dans un échantillon, comprenant les opérations suivantes :
i) mesure de troponine I dans ledit échantillon ;
ii) mesure d'un polypeptide à chaîne unique selon l'une quelconque des revendications 10 -13 dans un standard, ledit standard contenant une quantité connue de troponine I ;
iii) correlation de la troponine I mesurée dans l'échantillon avec la quantité connue de troponine I mesurée dans le standard afin de déterminer la quantité de troponine I dans ledit échantillon.


### SEQUENCE LISTING

<110> Shi, Qinwei
   Ling, Mingfu
   Liu, Shigui
<120> SINGLE-CHAIN POLYPEPTIDES COMPRISING TROPONIN I N-TERMINAL FRAGMENTS AND TROPONIN C
<130> 1112-1-071PCT
<140> PCT/IB00/01105
   <141> 2000-08-22
<160> 6
<170> Patent In Ver. 2.0
<210> 1
   <211> 744
   <212> DNA
   <213> Artificial Sequence
<220>
   <223> Description of Artificial Sequence: Synthetic derived from Homo Sapiens.
<400> 1
<210> 2
   <211> 247
   <212> PRT
   <213> Artificial Sequence
<220>
   <223> Description of Artificial Sequence: Synthetic derived from Homo Sapiens.
<400> 2
   Met Ala Tyr Ala Thr Glu Pro His Ala Lys Lys Lys Ser Lys Ile Ser
   1 5 10 15
   Ala Ser Arg Lys Leu Gln Leu Lys Thr Leu Leu Leu Gln Ile Ala Lys
   20 25 30
   Gln Glu Leu Glu Arg Glu Ala Glu Glu Arg Arg Gly Glu Lys Gly Arg
   35 40 45
   Ala Leu Ser Thr Arg Cys Gln Pro Leu Glu Leu Ala Gly Leu Gly Phe
   50 55 60
   Ala Glu Leu Gln Asp Leu Cys Arg Gln Leu His Ala Arg Val Asp Lys
   65 70 75 80
   Val Asp Glu Glu Ala Cys Met Asp Asp Ile Tyr Lys Ala Ala Val Glu
   85 90 95
   Gln Leu Thr Glu Glu Gln Lys Asn Glu Phe Lys Ala Ala Phe Asp Ile
   100 105 110
   Phe Val Leu Gly Ala Glu Asp Gly Cys Ile Ser Thr Lys Glu Leu Gly
   115 120 125
   Lys Val Met Arg Met Leu Gly Gln Asn Pro Thr Pro Glu Glu Leu Gln
   130 135 140
   Glu Met Ile Asp Glu Val Asp Glu Asp Gly Ser Gly Thr Val Asp Phe
   145 150 155 160
   Asp Glu Phe Leu Val Met Met Val Arg Cys Met Lys Asp Asp Ser Lys
   165 170 175
   Gly Lys Ser Glu Glu Glu Leu Ser Asp Leu Phe Arg Met Phe Asp Lys
   180 185 190
   Asn Ala Asp Gly Tyr Ile Asp Leu Asp Glu Leu Lys Ile Met Leu Gln
   195 200 205
   Ala Thr Gly Glu Thr Ile Thr Glu Asp Asp Ile Glu Glu Leu Met Lys
   210 215 220
   Asp Gly Asp Lys Asn Asn Asp Gly Arg Ile Asp Tyr Asp Glu Phe Leu
   225 230 235 240
   Glu Phe Met Lys Gly Val Glu
   245
<210> 3
   <211> 795
   <212> DNA
   <213> Artificial Sequence
<220>
   <223> Description of Artificial Sequence: Synthetic derived from Homo Sapiens.
<400> 3
<210> 4
   <211> 264
   <212> PRT
   <213> Artificial Sequence
<220>
   <223> Description of Artificial Sequence: Synthetic derived from Homo Sapiens.
<400> 4
   Met Ala Tyr Ala Thr Glu Pro His Ala Lys Lys Lys Ser Lys Ile Ser
   1 5 10 15
   Ala Ser Arg Lys Leu Gln Leu Lys Thr Leu Leu Leu Gln Ile Ala Lys
   20 25 30
   Gln Glu Leu Glu Arg Glu Ala Glu Glu Arg Arg Gly Glu Lys Gly Arg
   35 40 45
   Ala Leu Ser Thr Arg Cys Gln Pro Leu Glu Leu Ala Gly Leu Gly Phe
   50 55 60
   Ala Glu Leu Gln Asp Leu Cys Arg Gln Leu His Ala Arg Val Asp Lys
   65 70 75 80
   Val Asp Glu Glu Thr Ser Gly Gly Gly Gly Ser Gly Gly Gly Gly Ser
   85 90 95
   Gly Gly Gly Gly Ser Ala Cys Met Asp Asp Ile Tyr Lys Ala Ala Val
   100 105 110
   Glu Gln Leu Thr Glu Glu Gln Lys Asn Glu Phe Lys Ala Ala Phe Asp
   115 120 125
   Ile Phe Val Leu Gly Ala Glu Asp Gly Cys Ile Ser Thr Lys Glu Leu
   130 135 140
   Gly Lys Val Met Arg Met Leu Gly Gln Asn Pro Thr Pro Glu Glu Leu
   145 150 155 160
   Gln Glu Met Ile Asp Glu Val Asp Glu Asp Gly Ser Gly Thr Val Asp
   165 170 175
   Phe Asp Glu Phe Leu Val Met Met Val Arg Cys Met Lys Asp Asp Ser
   180 185 190
   Lys Gly Lys Ser Glu Glu Glu Leu Ser Asp Leu Phe Arg Met Phe Asp
   195 200 205
   Lys Asn Ala Asp Gly Tyr Ile Asp Leu Asp Glu Leu Lys Ile Met Leu
   210 215 220
   Gln Ala Thr Gly Glu Thr Ile Thr Glu Asp Asp Ile Glu Glu Leu Met
   225 230 235 240
   Lys Asp Gly Asp Lys Asn Asn Asp Gly Arg Ile Asp Tyr Asp Glu Phe
   245 250 255
   Leu Glu Phe Met Lys Gly Val Glu
   260
<210> 5
   <211> 57
   <212> DNA
   <213> Artificial Sequence
<220>
   <223> Description of Artificial Sequence: Linkers.
<400> 5
   actagtggtg gtggtggttc tggtgggggg ggttctggtg gcggtggttc tgcatgc 57
<210> 6
   <211> 19
   <212> PRT
   <213> Artificial Sequence
<220>
   <223> Description of Artificial Sequence: Linkers.
<400> 6
   Thr Ser Gly Gly Gly Gly Ser Gly Gly Gly Gly Ser Gly Gly Gly Gly
   1 5 10 15
   Ser Ala Cys